# EUROPÄISCHE PATENTSCHRIFT

(11) **EP 1 349 646 B1**
(45) Veröffentlichungstag und Bekanntmachung des Hinweises auf die Patenterteilung: **31.08.2005**
(21) Anmeldenummer: 01998393.1
(22) Anmeldetag: 17.11.2001
(51) Int. Cl.: B01D 71/02, A61M 1/14, A61M 1/16

(54) **WERKSTOFF ZUR DETOXIKATION KÖRPERFREMDER, TOXISCH WIRKENDER SUBSTANZEN IM BLUT**
MATERIAL FOR DETOXIFICATION OF BLOOD CONTAINING FOREIGN TOXIC SUBSTANCES
MATIERE PERMETTANT D'ELIMINER DES SUBSTANCES ETRANGERES TOXIQUES PRESENTES DANS LE SANG

(30) Priorität: 29.11.2000 DE 10059197
(43) Veröffentlichungstag der Anmeldung: 08.10.2003
(73) Patentinhaber: Intech Thüringen GmbH, 99880 Waltershausen (DE)
(72) Erfinder: SCHMAUDER, Hans-Peter, 07745 Jena (DE); SCHUNK, Werner, 99867 Gotha (DE); BRUDER, Michael, 22147 Hamburg (DE); KRAUSE, Karl-Heinz, 09123 Chemnitz (DE); MERKMANN, Gerhard, 99867 Gotha (DE)
(86) Internationale Anmeldenummer: PCT/DE2001/004335
(87) Internationale Veröffentlichungsnummer: WO 2002/043844

(56) Entgegenhaltungen:
- DD-A- 270 867
- DE-A- 2 722 025
- DE-A- 3 110 128
- DE-A- 3 738 691

## Beschreibung

Die Erfindung betrifft einen Werkstoff zur Detoxikation körperfremder, toxisch wirkender Substanzen im Blut, umfassend wenigstens:
- eine blutdurchlässige und blutverträgliche Matrix, insbesondere in Form einer Membran; sowie
- ein Molekularsieb, das in die Matrix eingearbeitet bzw. matrixmäßig verarbeitet und bei einem pH-Wert von 7,5 ± 0,5 mit einer chromatographierenden Lösung, insbesondere in Form einer Kationenlösung, beladen ist, wobei das chromatographierte Molekularsieb die Detoxikation des Blutes vornimmt.

Zur Blutreinigung werden verschiedene Verfahren eingesetzt, die alle das Ziel verfolgen, schädigende, toxische Substanzen aus dem Körper zu eliminieren. Neben der bekanntesten Methode, der Dialyse, gibt es die Hämofiltration und Hämodiafiltration, bei der das Blut durch eine mit einem Adsorber gefüllte Filterkerze gepumpt wird. Das Filtermaterial kann aus Hohlfasem (Kapillaren), Cellulose oder synthetischen Materialien, wie beispielsweise Polyamid, Polyacrylnitril, Polymethylmetacrylat oder Polysulfon, bestehen. Das Blut strömt dabei durch Poren dieses Filtermaterials (strukturierte Membran), das wiederum außen von einer Dialysierflüssigkeit umspült wird. Am Filtermaterial findet dann der Austausch der Blutsubstanzen statt. Diesbezüglich wird insbesondere auf die Patentschrift DD 270 867 A1 verwiesen.

Die Nephrologen und Dialysespezialisten fordern nun mehr und mehr eine individuelle Blutreinigung, insbesondere in Form der Dialyse.

Im Rahmen einer Weiterentwicklung besteht nun die Aufgabe der Erfindung darin, einen Werkstoff bereitzustellen, der der oben genannten Forderung nachkommt, verbunden mit einer größeren Wirksamkeit der Detoxikation bei gleichzeitiger Einhaltung des oben genannten pH-Wertes.

Zwecks Lösung dieser Aufgabe zeichnet sich nun der erfindungsgemäße Werkstoff dadurch aus, dass das chromatographierte Molekularsieb zusätzlich Kristallwasser enthält, und zwar derart, dass in Bezug auf eine Grundmolmenge (m) an Kristallwasser von wenigstens 100 das Molekularsieb bei einem Dehydratisierungsgrad von mindestens 40% partiell dehydratisiert ist, wobei das Molekularsieb mit der reduzierten Molmenge (m') an Kristallwasser mit der chromatographierenden Lösung beladen ist.

Das Molekularsieb ist insbesondere ein Metall-Aluminium-Silikat der folgenden Formel:

Meₙ [(AlO₂)ₓ · (SiO₂)_{Y}] · m(m')H₂O

Das Molekularsieb enthält dabei ein Grundmolmenge (m) an Kristallwasser von insbesondere wenigstens 200, das bei einem Dehydratisierungsgrad von vorzugsweise von 50 % bis 80 % partiell dehydratisiert ist.

Im Rahmen dieses Konzeptes ist vorzugsweise das Natrium-Aluminium-Silikat der folgenden Formel zu nennen:

Na₈₆ [(AlO₂)₈₆ · (SiO₂)₁₀₆] · 276 H₂O

Nach der partiellen Dehydratisierung ist das Molekularsieb mit der reduzierten Molmenge (m'; z.B. m' = 100) mit der Kationenlösung beladen, wobei dann das chromatographierte Molekularsieb in Wechselwirkung mit dem Blut seine detoxische Wirkung entfaltet.

Mit dem Einsatz eines partiell dehydratisierten Molekularsiebes als Träger für die Kationenlösung kann die Kationenzusammensetzung und Kationenkonzentration mittels des entsprechenden Dehydratisierungsgrades variabel unter Beachtung des pH-Wertes des Blutes von 7,5 ± 0,5 eingestellt werden.

Im Rahmen einer Bleientgiftung hat die Kationenlösung folgende Zusammensetzung:

| | |
|---|---|
| K⁺ | 4 bis 6 mmol/l |
| Ca²⁺ | 2 bis 3 mmol/l |
| Na⁺ | 130 bis 160 mmol/l |
| Fe²⁺ | 10 bis 40 mol/l |
| Mg²⁺ | 0,5 bis 1,5 mmol/l |
| NH₄⁺ | 10 bis 50 mol/l |
| Zn²⁺ | 5 bis 30 mol/l |

Die weiteren zweckmäßigen Werkstoffparameter sind:
- Der Beladungsgrad der chromatographierenden Lösung bzw. Kationenlösung ist kleiner als der Dehydratisierungsgrad des partiell dehydratisierten Molekularsiebes. Der Beladungsgrad ist dabei 45 % bis 75 %.
- Das chromatographierte Molekularsieb ist innerhalb der Matrix im Wesentlichen gleichmäßig verteilt.
- Die Matrix ist ein Polymerwerkstoff, insbesondere wiederum ein Elastomer, ein thermoplastisches Elastomer oder ein thermoplastischer Kunststoff. Wichtig in diesem Zusammenhang ist, dass diese Werkstoffe blutdurchlässig und blutverträglich sind.

Darüber hinaus besteht die Aufgabe darin, ein Verfahren zur Herstellung des erfindungsgemäßen Werkstoffes bereitzustellen.

Das Verfahren zur Herstellung des neuen Werkstoffes zeichnet sich nun durch folgende Verfahrensschritte aus:
- das Molekularsieb mit einer Grundmolmenge (m) an Kristallwasser von wenigstens 100 wird bei 400 bis 500°C, vorzugsweise bei etwa 450°C, mehrere Stunden, vorzugsweise 1 bis 7 Stunden, lang bei einem Dehydratisierungsgrad von mindestens 40% partiell dehydratisiert;
- anschließend wird das partiell dehydratisierte Molekularsieb mit der reduzierten Molmenge (m') an Kristallwasser mit der chromatographierenden Lösung sowie gegebenenfalls mit einem Antithrombotikum unter Bildung des chromatographierten Molekularsiebes beladen;
- schließlich wird das chromatographierte Molekularsieb in die Matrix eingearbeitet bzw. matrixmäßig verarbeitet.

Die Dehydratisierung und/oder Beladung wird/werden dabei insbesondere in Gegenwart eines inerten Gases, beispielsweise von Stickstoff, durchgeführt. Die Dehydratisierung und/oder Beladung erfolgt/erfolgen ferner vorzugsweise unter Normaldruck. Die Beladung selbst kann beispielsweise mittels einer Kugelmühle vorgenommen werden.

Die in der Beschreibung und in den Patentansprüchen erwähnten Aussagen zum Dehydratisierungsgrad und Beladungsgrad beziehen sich auf den Zustand nach Abschluss der partiellen Dehydratisierung bzw. der Beladung, d.h. ohne Stoffwechselmechanismus.

Die Erfindung wird nun anhand eines Ausführungsbeispiels unter Bezugnahme auf eine schematische Zeichnung erläutert.

Die Membran **1**, beispielsweise eine Dialyse-Membran, umfasst eine Matrix **2**, in die das chromotographierte Molekularsieb **3**, ein Addukt aus der Kationenlösung Y und dem Molekularsieb, eingearbeitet ist, wobei das Molekularsieb partiell dehydratisiert ist. Ferner ist das chromatographierte Molekularsieb innerhalb der Matrix im Wesentlichen gleichmäßig verteilt.

Im Blut **4** befindet sich nun die toxisch wirkende Substanz X, beispielsweise im Rahmen einer Bleivergiftung. Über die Grenzfläche **5** von Membran und Blut dringt die Substanz X in die Membran **2**. Mittels der Kationenlösung Y findet dabei eine Adsorption der toxisch wirkenden Substanz statt.

Gegebenenfalls ist das chromatographierte Molekularsieb zusätzlich mit einem Wirkstoff Z in Form eines Antithrombotikum beladen, wobei eine Adsorption von Wasser als Blutbestandteil unter Desorption des Wirkstoffes stattfindet. Im Zusammenhang mit der Verwendung eines partiell dehydratisierten Molekularsiebes kann über den Dehydratisierungsgrad die Abgabegeschwindigkeit des Wirkstoffes Z gesteuert werden. So wird beispielsweise ein Wirkstoff-Addukt mit einem Dehydratisierungsgrad von 60 % begieriger Wasser aufnehmen als ein Addukt mit einem Dehydratisierungsgrad von 30 %, und zwar in Bezug auf die gleiche Beladungsmenge an Wirkstoff.

Die Membran 1 kann beispielsweise in folgenden Formen auftreten, und zwar als:
- strukturierte Membran (porenartig, schaumartig);
- perforierte Membran;
- sorptionsblockartige Membran.

### Bezugszeichenliste

- **1**: Membran (Dialyse-Membran)
- **2**: Matrix
- **3**: chromatographiertes Molekularsieb
- **4**: Blut
- **5**: Grenzfläche von Membran und Blut
- X: körperfremde, toxisch wirkende Substanz im Blut
- Y: chromatographierende Lösung (Kationenlösung)
- Z: Wirkstoff (Antithrombotikum)

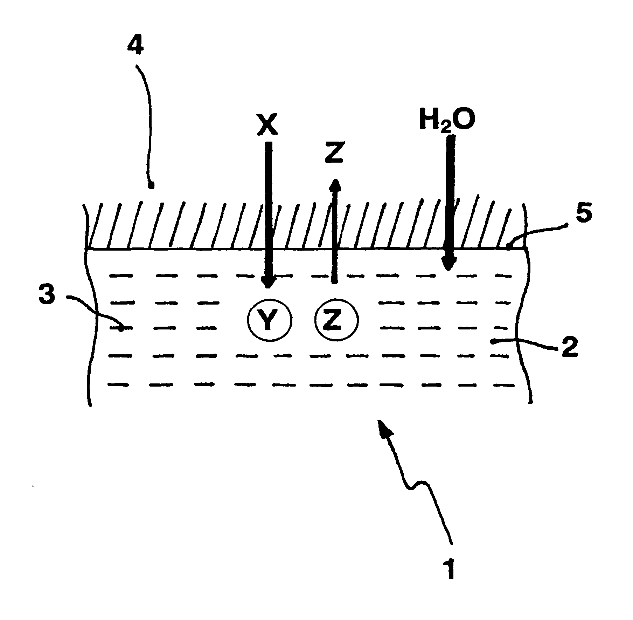

## Patentansprüche

1. Werkstoff zur Detoxikation körperfremder, toxisch wirkender Substanzen (X) im Blut (4), umfassend wenigstens:
- eine blutdurchlässige und blutverträgliche Matrix (2), insbesondere in Form einer Membran (1); sowie
- ein Molekularsieb, das in die Matrix (2) eingearbeitet bzw. matrixmäßig verarbeitet und bei einem pH-Wert von 7,5 ± 0,5 mit einer chromatographierenden Lösung (Y), insbesondere in Form einer Kationenlösung, beladen ist, wobei das chromatographierte Molekularsieb (3) die Detoxikation des Blutes (4) vornimmt;
**dadurch gekennzeichnet, dass**
- das chromatographierte Molekularsieb (3) zusätzlich Kristallwasser enthält, und zwar derart, dass in Bezug auf eine Grundmolmenge (m) an Kristallwasser von wenigstens 100 das Molekularsieb bei einem Dehydratisierungsgrad von mindestens 40% partiell dehydratisiert ist, wobei das Molekularsieb mit der reduzierten Molmenge (m') an Kristallwasser mit der chromatographierenden Lösung (Y) beladen ist.

2. Werkstoff nach Anspruch 1, **dadurch gekennzeichnet, dass** das Molekularsieb ein Metall-Aluminium-Silikat der folgenden Formel ist:
Meₙ [(AlO₂)ₓ · (SiO₂)_{Y}] · m' H₂O

3. Werkstoff nach Anspruch 2, **dadurch gekennzeichnet, dass** ein Metall der ersten oder zweiten Hauptgruppe des Periodensystems, vorzugsweise Natrium, Verwendung findet.

4. Werkstoff nach Anspruch 2 oder 3, **dadurch gekennzeichnet, dass** das Molekularsieb ein Natrium-Aluminium-Silikat der folgenden Formel ist:
Na₈₆ [(AlO₂)₈₆ · (SiO₂)₁₀₆] · m' H₂O

5. Werkstoff nach einem der Ansprüche 1 bis 4, **dadurch gekennzeichnet, dass** das Molekularsieb eine Grundmolmenge (m) an Kristallwasser von wenigstens 200 enthält.

6. Werkstoff nach einem der Ansprüche 1 bis 5, **dadurch gekennzeichnet, dass** das partiell dehydratisierte Molekularsieb einen Dehydratisierungsgrad von 50 % bis 80 % aufweist.

7. Werkstoff nach einem der Ansprüche 1 bis 6, **dadurch gekennzeichnet, dass** der Beladungsgrad der chromatographierenden Lösung (Y) kleiner ist als der Dehydratisierungsgrad des partiell dehydratisierten Molekularsiebes.

8. Werkstoff nach Anspruch 7, **dadurch gekennzeichnet, dass** der Beladungsgrad der chromatographierenden Lösung (Y) 45 % bis 75 % beträgt.

9. Werkstoff nach einem der Ansprüche 1 bis 8, **dadurch gekennzeichnet, dass** die chromatographierende Lösung (Y) eine Kationenlösung folgender Zusammensetzung ist:
Na⁺ K⁺ NH₄⁺ Ca²⁺ Mg²⁺ Fe²⁺ Zn²⁺

10. Werkstoff nach einem der Ansprüche 1 bis 9, **dadurch gekennzeichnet, dass** das chromatographierte Molekularsieb (3) innerhalb der Matrix (2) im Wesentlichen gleichmäßig verteilt ist.

11. Werkstoff nach einem der Ansprüche 1 bis 10, **dadurch gekennzeichnet, dass** die Matrix (2) ein Polymerwerkstoff, vorzugsweise ein Elastomer, ein thermoplastisches Elastomer oder ein thermoplastischer Kunststoff ist.

12. Werkstoff nach einem der Ansprüche 1 bis 11, **dadurch gekennzeichnet, dass** das chromatographierte Molekularsieb (3) zusätzlich mit einem Wirkstoff (Z) in Form eines Antithrombotikum beladen ist, so dass bei Blutkontakt der Matrix (2) eine Adsorption von Wasser unter Desorption des Wirkstoffes stattfindet, wobei der Kristallwassergehalt des Molekularsiebes zunimmt.

13. Verfahren zur Herstellung eines Werkstoffes nach einem der Ansprüche 1 bis 12, **gekennzeichnet durch** folgende Verfahrensschritte:
- das Molekularsieb mit einer Grundmolmenge(m) an Kristallwasser von wenigstens 100 wird bei 400 bis 500°C, vorzugsweise bei etwa 450°C, mehrere Stunden, vorzugsweise 1 bis 7 Stunden, lang bei einem Dehydratisierungsgrad von mindestens 40% partiell dehydratisiert;
- anschließend wird das partiell dehydratisierte Molekularsieb mit der reduzierten Molmenge (m') an Kristallwasser mit der chromatographierenden Lösung (Y) sowie gegebenenfalls mit einem Antithrombotikum unter Bildung des chromatographierten Molekularsiebes (3) beladen;
- schließlich wird das chromatographierte Molekularsieb (3) in die Matrix (2) eingearbeitet bzw. matrixmäßig verarbeitet.

14. Verfahren zur Herstellung eines Werkstoffes nach Anspruch 13, **dadurch gekennzeichnet, dass** die Dehydratisierung und/oder Beladung in Gegenwart eines inerten Gases, beispielsweise von Stickstoff, durchgeführt wird/werden.

15. Verfahren zur Herstellung eines Werkstoffes nach Anspruch 13 oder 14, **dadurch gekennzeichnet, dass** die Dehydratisierung und/oder Beladung unter Normaldruck durchgeführt wird/werden.

## Claims

1. Material for detoxification of exogenous substances (X) with a toxic effect in the blood (4), comprising at least:
- a blood-permeable and blood-compatible matrix (2), in particular in the form of a membrane (1), and
- a molecular sieve which is incorporated in the matrix (2) or processed in matrix form and is charged with a chromatographic solution (Y), in particular in the form of a cationic solution, at a pH value of 7.5+0.5, the chromatographed molecular sieve (3) carrying out the detoxification of the blood (4),
**characterised in that**
- the chromatographed molecular sieve (3) additionally contains water of crystallisation, such that the molecular sieve is partially dehydrated with a degree of dehydration of at least 40% in relation to a basic molar quantity (m) of water of crystallisation of at least 100, the molecular sieve with the reduced molar quantity (m') of water of crystallisation being charged with the chromatographic solution (Y).

2. Material according to claim 1, **characterised in that** the molecular sieve is a metal-aluminium silicate with the following formula:
Meₙ[(AlO₂)ₓ . (SiO₂)_{y}] . m'H₂O

3. Material according to claim 2, **characterised in that** a metal of the first or second main group of the periodic system, preferably sodium, is used.

4. Material according to claim 2 or 3, **characterised in that** the molecular sieve is a sodium-aluminium silicate with the following formula:
Na₈₆[(AlO₂)₈₆ . (SiO₂)₁₀₆] . m'H₂O

5. Material according to one of claims 1 to 4, **characterised in that** the molecular sieve contains a basic molar quantity (m) of water of crystallisation of at least 200.

6. Material according to one of claims 1 to 5, **characterised in that** the partially dehydrated molecular sieve exhibits a degree of dehydration of 50% to 80%.

7. Material according to one of claims 1 to 6, **characterised in that** the degree of charging of the chromatographic solution (Y) is less than the degree of dehydration of the partially dehydrated molecular sieve.

8. Material according to claim 7, **characterised in that** the degree of charging of the chromatographic solution (Y) is 45% to 75%.

9. Material according to one of claims 1 to 8, **characterised in that** the chromatographic solution (Y) is a cationic solution with the following composition:
Na⁺ K⁺ NH₄⁺ Ca²⁺ Mg²⁺ Fe²⁺ Zn²⁺

10. Material according to one of claims 1 to 9, **characterised in that** the chromatographed molecular sieve (3) is essentially distributed uniformly inside the matrix (2).

11. Material according to one of claims 1 to 10, **characterised in that** the matrix (2) is a polymer material, preferably an elastomer, a thermoplastic elastomer or a thermoplastic plastic.

12. Material according to one of claims 1 to 11, **characterised in that** the chromatographed molecular sieve (3) is additionally charged with an active substance (Z) in the form of an anti-thrombotic, so that when the matrix (2) comes into contact with the blood, adsorption of water and desorption of the active substance take place, and the content of water of crystallisation of the molecular sieve increases.

13. Method for manufacturing a material according to one of claims 1 to 12, **characterised by** the following process steps:
- the molecular sieve with a basic molar quantity (m) of water of crystallisation of at least 100 is partially dehydrated at 400 to 500°C, preferably at approximately 450°C, for a plurality of hours, preferably 1 to 7 hours, with a degree of dehydration of at least 40%;
- then the partially dehydrated molecular sieve with the reduced molar quantity (m') of water of crystallisation is charged with the chromatographic solution (Y) and possibly with an anti-thrombotic forming the chromatographed molecular sieve (3);
- finally, the chromatographed molecular sieve (3) is incorporated in the matrix (2) or processed in matrix form.

14. Method for manufacturing a material according to claim 13, **characterised in that** the dehydration and/or charging is carried out in the presence of an inert gas, for example nitrogen.

15. Method for manufacturing a material according to claim 13 or 14, **characterised in that** the dehydration and/or charging is carried out at normal pressure.

## Revendications

1. Matériau pour la détoxication de substances (X) exogènes à effet toxique dans le sang (4), comprenant au moins :
- une matrice (2), perméable au sang et tolérée par le sang, en particulier sous forme d'une membrane (1) ; et aussi
- un tamis moléculaire, qui est incorporé dans la matrice (2) ou est mis en oeuvre à la manière d'une matrice, et qui, à un pH de 7,5 ± 0,5, est chargé d'une solution (Y) à chromatographier, en particulier sous forme d'une solution de cations, le tamis moléculaire chromatographié (3) assurant la détoxication du sang (4) ;
**caractérisé en ce que**
- le tamis moléculaire chromatographié (3) contient en outre de l'eau de cristallisation, et plus précisément de telle sorte que, par rapport à une quantité molaire de base (m) d'eau de cristallisation d'au moins 100, le tamis moléculaire soit partiellement déshydraté pour un degré de déshydratation d'au moins 40 %, le tamis moléculaire contenant la quantité molaire réduite (m') d'eau de cristallisation étant chargé de la solution à chromatographier (Y).

2. Matériau selon la revendication 1, **caractérisé en ce que** le tamis moléculaire est un silicate de métal et d'aluminium ayant la formule suivante :
Meₙ[(AlO₂)ₓ . (SiO₂)_{y}] . m'H₂O

3. Matériau selon la revendication 2, **caractérisé en ce qu'**on utilise un métal du premier ou du deuxième groupe principal du Tableau Périodique, de préférence le sodium.

4. Matériau selon la revendication 2 ou 3, **caractérisé en ce que** le tamis moléculaire est un silicate de sodium et d'aluminium ayant la formule suivante :
Na₈₆[(AlO₂)₈₆ . (SiO₂)₁₀₆] . m'H₂O

5. Matériau selon l'une des revendications 1 à 4, **caractérisé en ce que** le tamis moléculaire contient une quantité molaire de base (m) d'eau de cristallisation d'au moins 200.

6. Matériau selon l'une des revendications 1 à 5, **caractérisé en ce que** le tamis moléculaire partiellement déshydraté a un degré de déshydratation de 50 à 80%.

7. Matériau selon l'une des revendications 1 à 6, **caractérisé en ce que** le degré de charge de la solution à chromatographier (Y) est inférieur au degré de déshydratation du tamis moléculaire partiellement déshydraté.

8. Matériau selon la revendication 7, **caractérisé en ce que** le degré de charge de la solution à chromatographier (Y) est de 45 à 75 %.

9. Matériau selon l'une des revendications 1 à 8, **caractérisé en ce que** la solution à chromatographier (Y) est une solution de cations ayant la composition suivante :
Na⁺ K⁺ NH₄⁺ Ca²⁺ Mg²⁺ Fe²⁺ Zn²⁺

10. Matériau selon l'une des revendications 1 à 9, **caractérisé en ce que** le tamis moléculaire à chromatographier (3) est réparti d'une manière essentiellement uniforme dans la matrice (2).

11. Matériau selon l'une des revendications 1 à 10, **caractérisé en ce que** la matrice (2) est un matériau polymère, de préférence un élastomère, un élastomère thermoplastique ou un matériau plastique thermoplastique.

12. Matériau selon l'une des revendications 1 à 11, **caractérisé en ce que** le tamis moléculaire à chromatographier (3) est en outre chargé d'un principe actif (Z) sous forme d'un antithrombotique, de telle sorte que, après contact de la matrice (2) avec le sang, il se produise une adsorption d'eau avec désorption du principe actif, avec augmentation de la teneur du tamis moléculaire en eau de cristallisation.

13. Procédé de préparation d'un matériau selon l'une des revendications 1 à 12, **caractérisé par** les étapes suivantes :
- le tamis moléculaire, qui présente une quantité molaire de base (m) d'eau de cristallisation d'au moins 100, est soumis à une déshydratation partielle à une température de 400 à 500°C, de préférence d'environ 450°C pendant plusieurs heures, de préférence pendant 1 à 7 heures, pour un degré de déshydratation d'au moins 40 % ;
- puis le tamis moléculaire partiellement déshydraté, contenant la quantité molaire réduite (m') d'eau de cristallisation, est chargé de la solution à chromatographier (Y) et éventuellement d'un antithrombotique avec formation du tamis moléculaire chromatographié (3) ;
- finalement, le tamis moléculaire chromatographié (3) est incorporé dans la matrice (2) ou est mis en oeuvre à la manière d'une matrice.

14. Procédé de préparation d'un matériau selon la revendication 13, **caractérisé en ce que** la déshydratation et/ou la charge sont effectuées en présence d'un gaz inerte, par exemple l'azote.

15. Procédé de préparation d'un matériau selon la revendication 13 ou 14, **caractérisé en ce que** la déshydratation et/ou la charge sont réalisées sous la pression normale.
